Europäisches Patentamt

European Patent Office

Office européen des brevets

(11) Numéro de publication : **0 409 690 B1**

(12) **FASCICULE DE BREVET EUROPEEN**

(45) Date de publication du fascicule du brevet :
08.09.93 Bulletin 93/36

(51) Int. Cl.⁵ : **A61K 7/00, A61K 9/51**

(21) Numéro de dépôt : **90401957.7**

(22) Date de dépôt : **06.07.90**

(54) **Produits pour application cutanées, à effets cosmétiques et/ou thérapeutiques.**

Le dossier contient des informations techniques présentées postérieurement au dépôt de la demande et ne figurant pas dans le présent fascicule.

(30) Priorité : **18.07.89 FR 8909603**

(43) Date de publication de la demande :
**23.01.91 Bulletin 91/04**

(45) Mention de la délivrance du brevet :
**08.09.93 Bulletin 93/36**

(84) Etats contractants désignés :
**BE DE ES GB GR IT NL**

(56) Documents cités :
**EP-A- 0 274 961**
**EP-A- 0 316 054**
**EP-A- 0 352 190**
**WO-A-87/07150**
**GB-A- 2 166 651**

(73) Titulaire : **EXSYMOL S.A.M.**
**4 Avenue Prince Héréditaire Albert**
**MC-98000 Monte Carlo (MC)**

(72) Inventeur : **Bommelaer, Jean**
**123 Boulevard Jacques Monod**
**F-06010 Le Cannet (FR)**
Inventeur : **Franco, André**
**9 Avenue Laurenti**
**F-06500 Menton (FR)**
Inventeur : **Gueyne, Jean, Périgord 1**
**6 Lacets Saint-Léon, Monte-Carlo**
**MC-98000 Monaco (MC)**
Inventeur : **Seguin, Marie-Christine, Périgord 1**
**6 Lacets Saint-Léon, Monte-Carlo**
**MC-98000 Monaco (MC)**

(74) Mandataire : **Kohn, Armand c/o S.A.**
**FEDIT-LORIOT ET AUTRES et al**
**CONSEILS EN PROPRIETE INDUSTRIELLE 38,**
**avenue Hoche**
**F-75008 Paris (FR)**

## Description

L'invention concerne des nouveaux produits pour application cutanée, susceptibles d'exercer un effet cosmétique, thérapeutique ou, à la fois, cosmétique et thérapeutique, suivant un certain ordre dans le temps. Ces produits apportent un progrès marqué dans le domaine de l'action sur ou par la peau ; notamment, ils permettent d'éviter les surcharges en substances, souvent inutiles ou même nocives, fréquentes lors des traitements classiques. Un important avantage de l'invention réside en ce qu'elle permet de réaliser une action chronobiologique dont on connaît, depuis quelque temps, les effets favorables. Il apparaît, d'après des études de ces dernières années, que l'efficacité d'un agent thérapeutique dépend de l'heure de son application : elle varie selon le moment du cycle physiologique quotidien, de l'individu. Or, les nouveaux produits suivant l'invention se prêtent bien à une application permettant de déterminer d'avance leur action dans le temps. Une seule application d'un tel produit peut permettre l'obtention de ses effets à un ou plusieurs moments de la journée ou de la nuit. Grâce à la nature particulière du véhicule, utilisé pour les susbtances actives, un produits selon l'invention peut être employé en quantité totale bien moindre de celle qui est nécessaire dans les cas des produits habituels, notamment pommades, crèmes, solutions, poudres, etc.

Le produit suivant l'invention, qui contient une ou plusieurs substances actives, portées par des microsphères d'un polymère, dispersées dans un liquide non solvant de ce polymère, et qui comprend au moins deux sortes d'ensembles microsphères-substance active, libérant la substance active en des temps différents, est caractérisé en ce que la ou les substances actives sont fixées aux microsphères par des forces de liaison différentes.

Les microsphères de différents polymères, et la technique de leur préparation sont connues, il n'y a donc pas lieu de les décrire ici. On peut trouver la description de tels produits et de leur mode de préparation, par exemple, dans les brevets FR 1 572 106 et 2 304 326, EP 0 064 967 ou EP 0 274 961 ; mais il n'y est pas question de réaliser un effet chrono-biologique comme dans la présente invention.

Bien que l'invention puisse porter sur des sphérules microscopiques de diverses grosseurs, de préférence les dimensions ne dépassent pas 1000 nm, les tailles préférées se rangent entre 50 et 500 nm ou, mieux encore, entre 60 et 300 nm.

L'optimum de la grosseur des particles dépend d'ailleurs de la nature du polymère qui les constitue.

Etant donné la grande finesse des microsphères utilisées suivant l'invention, leur surface spécifique par unité de poids est considérable, d'où adsorption ou combinaison électrostatique ou covalente très poussée des substances actives mises en contact de ces particules ; il s'en suit, comme mentionné plus haut, la possibilité d'avoir des produits beaucoup plus riches en substances actives que ne le permettent les compositions habituelles qui nécessitent des fortes proportions d'excipient dont un premier défaut, non le moindre, est le bouchage des pores de la peau. Comme la substance active, dans les produits suivant l'invention, est liée aux microsphères, elle n'est libérée que progressivement et - par conséquent - ne risque pas d'occasionner de surdosage, tout en se trouvant potentiellement en quantité pouvant être relativement très forte.

Les microparticules, convenant à la réalisation de l'invention, peuvent être choisies parmi les diverses microsphères en polymères connus, pleines ou creuses, à condition d'être insolubles dans le liquide véhicule utilisé. Celui-ci est d'ailleurs, le plus souvent, constitué par de l'eau ; il peut néanmoins être un liquide organique toléré par la peau et l'organisme, tel que par exemple un polyol comme glycol ou glycérine, un éther ou ester de polyol, notamment un corps gras, en particulier huile végétale ou animale, biologiquement acceptable.

Ainsi, conviennent à l'application suivant l'invention des dispersions de microparticules en des polymères comme polysaccharides, polyamides, polyalkylènes, polyarylalkylènes, polyalkylidènes, polysilicones et autres ; dans chacune de ces classes nombreux dérivés et copolymères peuvent être employés. Par exemple, on peut utiliser des polysaccharides tels que xanthane, scléroglucane, pectines, amidons, celluloses, cyclodextrines et leurs dérivés comme amylose, amylopectine, carboxyméthyl-cellulose, hydroxy-cellulose, alkylcelluloses, dextrine, etc. Des polysaccharides polymérisés avec des protéines peuvent être utilisés lorsqu'on désire en même temps la microencapsulation des substances actives.

En tant que polyaryl-alkylènes, on peut citer le polystyrène et surtout ses copolymères, en particulier avec des esters éthyléniques, notamment acrylates ou méthacrylates de différents alkyles, d'hydroxy-alkyles, avec du méthacryl- ou acrylamide. De même, conviennent les résines vinyliques, par exemple l'acétate de polyvinyle et ses copolymères avec des acrylates ou méthacrylates, etc.

Le pouvoir d'adsorption et l'affinité vis-à-vis des substances actives cosmétiques ou thérapeutiques varient suivant la nature du polymère constituant les microsphérules et avec les dimensions de celles-ci ; ces faits sont mis à profit dans la présente invention pour obtenir des produits plus ou moins chargés en substances actives et libérant ces dernières en un temps plus ou moins long. Aussi, suivant un trait particulier de l'invention, des produits, libérant les substances actives en des temps différents, sont caractérisés par la présence conjointe de plusieurs types de microsphères de dimensions ou/et natures de polymère différentes.

2

Par exemple, un produit qui, appliqué le matin, doit agir en trois temps différents au cours de la journée, comprend en dispersion aqueuse trois sortes de microsphères ayant 90 à 160 nm de diamètre ; 1° en polysiloxane, 2° en polyadipamide et 3° en copolymère styrène (90)-méthacrylate de méthyle (10).

Dans une autre variante de l'invention, un produit cosmétique que l'on veut faire agir sur la peau de façon progressive et échelonnée dans le temps, après une application unique, comprend par exemple - en tant que microsphères en copolymère de méthacrylate de butyle (70)/méthacrylamide (30) - 1,1 % en poids de ces microsphères dont 32% présentent des dimensions de 60 à 100 nm, 45% des dimensions de 150 à 250 nm et 23% des diamètres d'environ 300 à 500 nm.

Une autre forme d'exécution de l'invention consiste en une suspension de microsphères auxquelles une seule ou plusieurs substances actives sont fixées par des forces de liaison différentes. Ainsi peut-on avoir, suivant l'invention, une suspension de sphères microscopiques d'un polymère surlesquelles une substance active est adsorbée, une autre est liée au polymère par des liaisons de nature chimique, une troisième substance pouvant éventuellement adhérer aux sphères par des liaisons électrostatiques ou ioniques. Il est possible d'avoir une seule substance active, liée aux sphères à la fois par adsorption et par des liaisons chimiques ou/et électrostatiques.

Avec certaines substances actives et avec des polymères appropriés, les sphères suivant l'invention peuvent absorber la substance, formant ainsi une sorte de solution solide.

Grâce à cette différenciation des liaisons les temps de libération de la ou des substances actives sont également différenciés, ce qui permet de produire un effet chrono-biologique par l'utilisation des suspensions suivant l'invention.

Il est à noter que, dans le cas des liaisons covalentes entre la substance active et les microsphères de polymère, ces liaisons sont directes ou indirectes ; ces dernières comprennent une molécule intermédiaire qui assure la jonction entre la substance active et des groupes fonctionnels du polymère. De telles molécules intermédiaires peuvent être, selon les cas, celles de composés porteurs de deux fonctions comme par exemple diacides, diamines, amino-acides, dialdéhydes telles que glyoxal, glutaraldéhyde, glyoxime, des carbodiimides , etc. Dans ce cas, le procédé de préparation de la suspension aqueuse ou organique des microsphères comprend en général l'addition du composé intermédiaire avant l'adjonction de la substance active que l'on veut fixer au moyen de ce composé intermédiaire.

Lorsque les microsphères sont creuses, elles sont - co-formément à l'invention - en même temps adsorbantes ou/et porteuses de groupes fonctionnels : elles permettent une action chrono-biologique, car une ou plusieurs substances actives encapsulées, sont relâchées en des temps différents par les creux des sphères, par les forces d'adsorption et par les diverses liaisons chimiques et physico-chimiques des substances avec le polymère.

Un mode de liaison, qui s'ajoute souvent aux précédents, est l'absorption dans les pores que peuvent présenter les sphères ; cela apporte une différenciation de plus des temps de libération de la substance active.

De façon générale, les produits suivant l'invention contiennent en poids 0,1 à 10%, et plus souvent 0,3 à 3%, des microparticules définies plus haut, la teneur choisie dépendant de la nature du ou des polymères, de la taille des particules, de leur affinité vis-à-vis des substances actives et des nature et proportion du liquide de la dispersion.

En ce qui concerne les substances actives, leur quantité est de préférence celle qui peut être fixée au maximum par les microsphères ; cette fixation qui - comme on a vu - peut avoir lieu par adsorption, liaison covalente ou autre combinaison physico-chimique, ou/et par encapsulation dans le cas de microbilles creuses, porte de préférence sur la majeure partie du poids des substances actives. S'il n'y a pas de contre-indication biologique, voire médicale, un certain excès de cette ou ces substances peut préexister, sans inconvénient, en solution ou suspension dans le liquide du produit. D'une façon générale, suivant les cas, la teneur en substances actives, dans le produit selon l'invention, est de 0,01 à 90% en poids. Cette large gamme se justifie par le fait que certains corps actifs, comme par exemple hormones, vitamaines ou enzymes peuvent être utilisés à très faible dose, tandis que d'autres, tels que réhydratants, antirides ou régénérants gagnent à être employés en assez fortes quantités.

Parmi les différentes substances actives cosmétiquement, on peut citer non limitativement des hydratants, préventifs, régénérateurs, réparateurs, amincissants, antiradicaux libres, antiglyclosylants non enzymatiques, protecteurs contre les rayons ultraviolets, activateurs de bronzage, colorants, désodorisants. Ainsi, font par exemple objet de l'invention des produits dans lesquels des microsphères, définies plus haut, portent des substances comme hyaluronate de diméthyl-silangié (D.S.H.C.), combinaisons de protéine avec un silanol, théophylline, caféine, tyrosine, silanol-tyrosine, extrait de camomille, acide hyaluronique, collagène, élastine partiellement hydrolysée, théobromine, corps gras et bien d'autres.

En tant que substances actives, thérapeutiques, ou à la fois thérapeutiques et cosmétiques, le produit suivant l'invention peut renfermer des vitamines, des hormones, des enzymes, des vasodilatateurs ou vasocons-

3

trictifs, des anti-inflammatoires, antiseptiques, cholagogues, diurétiques, antiallergiques, neuroleptiques, etc. C'est ainsi que des microsphères ont adsorbé ou fixé des substances comme par exemple vitamines A, $B_1$, $B_2$,$B_6$, C, $D_3$, E, K, PP, oestrogène, androstane, extrait de Fragon (Ruscus Aculeatus), escine, acide acétylsalicylique, glafénine, esculoside, dextro-propoxyphène (HCl), pipérazine, diazépam, oxazepam, prométazine, etc.

Pour que la ou les substances actives se fixent sur les microparticules par au moins un des mécanismes évoqués plus haut,il est préférable qu'elles soient à l'état liquide, en solution ou en suspension fine, mouillant les particules. Cela est particulièrement recommandable lorsque le polymère a peu d'affinité pour le milieu aqueux, comme c'est le cas de microbilles en polystyrène ou en copolymère riche en styrène, ou lorsque les microsphères ne contiennent pas ou pas assez de tensioactif provenant de leur préparation. Suivant une variante de l'invention, on incorpore au produit au moins un composé auxiliaire, liquide, miscible avec le liquide de dispersion des microsphères, pour améliorer le contact entre les substances actives et les microsphères.

Lorsque les substances actives se fixent sur ces dernières par adsorption, la masse moléculaire du composé auxiliaire doit être inférieure à celle de la substance active. Conviennent, en tant que composés auxiliaires des polyols, des polysaccharides et mucopolysaccharides solubles ou gonflables à l'eau, et leurs dérivés siliciés, lécithine, ou/et surtout des composés tensioactifs, par exemple tels que polyoxyéthylène, esters gras polyoxyéthylénés du sorbital et autres tensioactifs tolérés par la peau. Il existe des substances actives qui peuvent également servir de composé auxiliaire ; c'est par exemple le cas des esters de l'acide hyaluronique et notamment celui de diméthyl silane diol qui, tout en servant de réhydratant énergique , peut faciliter la fixation de diverses molécules sur des microsphères. La teneur en composé auxiliaire peut varier entre 0,1 et 80%.

La préparation du produit suivant l'invention peut s'effectuer par la mise en suspension de la quantité voulue de microbilles dans un liquide de dispersion, le plus souvent eau, glycol ou alcool, dans un liquide devant servir de substance active, ou dans le mélange de ces deux liquides. L'opération a lieu à la température ambiante, sous légère agitation. Eventuellement, au cours de celle-ci on ajoute un composé auxiliaire.

Le produit obtenu est conservé de préférence à une température entre l'ambiante et 1°C, les températures inférieures à 0°C risquant d'en provoquer l'altération.

L'invention est illustrée, sans limitation, par les exemples qui suivent.

## EXEMPLE 1

Produit cosmétique réhydratant sous la forme de nanosphères de type classique.

On mélange 90 g d'une solution aqueuse de hyaluronate de diméthyl silane diol, à 19,5 g/kg, c'est-à-dire 0,9 g/kg en Si, avec 5 g de microsphères d'un copolymère de 60% en poids de styrène avec 40% de méthacrylate de méthyle, de diamètres 65 à 125 nm (moyenne 95 nm), en suspension dans 9,5 g d'eau, agité pendant 10 minutes à la température ambiante, puis laissé au repos pendant 48 heures.
On constate que les microsphères se sont chargées de 30% de hyaluronate de diméthylsilanediol.

## EXEMPLE 2

Comparatif pour les exemples 3 à 9.

Produit régénérant, pour la peau, à base d'une combinaison de silanol et d'élastine (élastinate de silanol)

Des microsphères de copolymère de 4 moles de styrène avec 1 mole d'acide acrylique sont préparées par le procédé connu, qui consiste à polymériser une émulsion aqueuse de ces deux monomères, l'émulsifiant étant le dodécylsulfate de Na et le catalyseur le persulfate de K. Après polymérisation, neutralisation de la suspension formée, sa purification par dialyse à l'eau et passage sur une résine échangeuse d'ions, 10 ml de la suspension contiennent $10^{16}$ sphérules du polymère, soit 0,55 g, d'une taille moyenne de 100 nm. 10 ml de cette suspension sont ajoutés à 90 ml d'une solution aqueuse d'élastinate de silanol à 46 g/kg soit 1,8 g Si. Après séparation des microsphères, on constate qu'elles ont adsorbé 28% de la combinaison élastine-silanol, soit une quantité renfermant 0,504 g de Si. Le produit, ainsi obtenu, est utilisé, dispersé dans une crème cosmétique courante, sous trois formes : à raison de 0,2, de 2 et 5% en poids. Dans tous les cas, il y a un effet net de régénération ; la combinaison élastine-Si est libérée en des temps de 8 à 15 heures par les microsphères (déterminé à l'aide d'une cellule de Frantz).

## EXEMPLE 3

Après avoir opéré comme dans l'exemple 2, on remet les 5,5 g de microsphères séparées en dispersion dans 55 ml d'une émulsion composée de : 20 ml d'eau, 25 ml d'huile d'amandes douces, 8 ml de polyéthylène-glycol, 1 ml de glycérine et 1 ml de laurate de polyoxyéthylène-sorbitol. Une seule application quotidienne suffit pour produire des effets marqués de régénération de la peau.

## EXEMPLE 4

Produit comprenant deux substances actives agissant en des temps différents

Les microsphères sont constituées par un copolymère de 2 moles de styrène avec 2 moles de butadiène et 1 de méthacrylate d'amino-2 éthanol ; elles ont été préparées à la manière connue,en dispersion aqueuse avec du dodécylsulfate de Na comme tensioactif et persulfate de K en tant que catalyseur. Après polymérisation, les microsphères sont neutralisées, purifiées par dialyse aqueuse et passées sur une résine échangeuse d'ions.

A 100 ml de suspension de 4 g de ces microsphères au nombre de $4 \times 10^4$ environ (taille moyenne 96 nm) on ajoute 0,09 g d'acide pyrrolidone carboxylique qui se lie à la fonction-$NH_2$ du méthacrylate, formant une liaison covalente amidique. On procède alors à une nouvelle purification, et la suspensionest additionnée de 6,1 g de hyaluronate de diméthyl silanol, le même qu'à l'exemple 1, qui est adsorbé.

La suspension résultante de 106,1 g comprend ainsi 0,09 g d'acide pyrolidone carboxylique et 0,28 g de Si du hyaluronate de diméthyl silanol ; l'application à la peau du produit tel que, ou mélangé à une crème cosmétique, gel ou lait, conduit à la libération du composé pyrrolidénique en 2 à 5 heures et à celle du hyaluronate en 10 à 17 heures. On assiste ainsi à une action synergique des deux substances actives pendant 2 à 5 heures, suivie d'une période plus longue de l'action du hyaluronate seul. Cela se traduit par des effets de réhydratation extrèmement favorables.

## EXEMPLE 5

Produit dans lequel des microsphères portent trois substances actives différentes

Les microsphères, préparées à la mnière connue, rappelée en tête de l'exemple 4, sont constituées par un copolymère de 4 moles de styrène avec 1 mole d'acide acrylique.

(A) - Sur 100 g de suspension aqueuse de 5g de microsphères on fait réagir 0,126 g de théophylline qui se fixe aux groupes acryliques.

(B) - Les microsphères sont alors fonctionnalisées au moyen d'hexaméthylène diamine, après quoi on les traite avec une solution aqueuse à 40g/kg d'élastine : 0,3 g de polypeptides d'élastine se fixent ainsi sur le copolymère.

(C) - On purifie le produit obtenu et on lui fait adsorber des glucosides du Fragon, à partir d'un extrait aqueux de cette plante (Ruscus 1=33).

Le produit final, en dispersion aqueuse ou huileuse à 20% se prête bien aux applications cutanées et exerce une action amincissante, antirétention d'eau, stimulante de la microcirculation superficielle lymphatique et veineuse, ainsi que rafermissante de la peau.

Il convient bien à l'incorporation dans divers cosmétiques connus, tels que crèmes,gels, laits, etc., de préférence à raison de 1 à 7% en poids.

Les trois substances actives agissent pendant des temps différents : l'élastine en 8 à 19 heures après l'application; les glucosides du Ruscus durant 24 heures ; la théophylline en 2 à 8 heures.

## EXEMPLE 6

Des microsphères ont été préparées à partir d'un copolymère de 2 moles de styrène , 2 moles d'acétate de vinyle et 1 mole d'acide méthacrylique. A 100 g de suspension aqueuse de 3 g de ces microsphères, de 120 nm de taille moyenne, on a fixé 0,12 g de tyrosine.

Les microsphères sont ensuite traitées par de l'hexaméthylène diamine, y attachant ainsi une fonction amine. Après purification on leur fait adsorber les deux filtres solaires : acide sulfonique hydroxy-2 méthoxy-4-benzophénone-5 et le salicylate d'éthyl-2 hexyle. Incorporés dans des crèmes solaires courantes, à raison de 1 à 7%, ces produits, en une seule application dans la journée, améliorent considérablement la protection contre les UV, A et B, grâce à l'allongement de la diffusion progressive des deux filtres.

EXEMPLE 7

Modification des durées d'action de deux substances actives, par leur fixation selon deux mécanismes différents sur les mêmes nanosphères.

On prépare 100 g d'une suspension aqueuse de 5 g de nanosphères de diamètre d'environ 70 à 130 nm (moyenne 100 nm) par la copolymérisation d'une émulsion de 4 moles de styrène avec 1 mole d'acide acrylique, suivant la technique de l'exemple 2. A ces 100 g, on ajoute 0,126 g de théophylline qui se fixe aux groupes acryliques acides du copolymère par interaction chimique du type électronique.

A l'ensemble, ainsi obtenu, on ajoute 0,192 g d'une combinaison de 1 mole de théophylline avec 1 mole de $CH_3Si(OH)_3$, désignée ci-après par le terme "théophyllisilane C" de composition globale $C_7H_8N_4O_2.CH_3-Si(OH)_3$. Après 1 heure d'agitation, on trouve - par ultracentrifugation - que 28% de la quantité utilisée de théophyllisilane a été adsorbée par les nanosphères.

On est ainsi en présence d'un produit liquide (a-b) composé de deux ensembles nanosphères de copolymère/substance active :

(a) - 0,126 g de théophylline fixée chimiquement à 5 g de nanosphères dans 100 g de liquide aqueux ;
(b) - 0,0538 g de théophyllisilane C adsorbé par les 5 g des mêmes nanosphères dans les 100 g de liquide aqueux ;

le liquide aqueux tient en solution 0,1382 g de théophylli-silane C (72% des 0,192 g mis en oeuvre).

Le liquide (a-b) est soumis à des essais sur un modèle expérimental, reproduisant les phénomènes de diffusion dans la peau.

On détermine ainsi les proportions de chacune des substances actives qui auraient pu être au contact de la peau en 3 heures. D'autre part, des déterminations similaires sont effectuées sur une solution aqueuse (cd) contenant, dans 100 g, 0,126 g de théophylline et 0,192 g de théophyllisilane C, sans aucune particule de polymère.

Voici les % de la quantité initiale de chacune de ces substances actives, qui, d'après les essais susmentionnés, auraient pu diffuser dans la peau à partir du liquide étudié :

|  | (a-b) (avec nanosphères) | (c-d) (sans nanosphères) |
|---|---|---|
| Théophylline | 10% | 25% |
| Théophyllisilane C | 20% | 35% |

Cela signifie que la mise à la disposition de la peau, de substances actives, est nettement tempérée, lorsque ces substances sont utilisées sous la forme (a-b) suivant l'invention : 25 : 10 = 2,5 fois pour la théophylline et 35 : 20 = 1,75 fois pour le théophyllisilane. Et il devient possible d'avoir encore, à partir de la même dose appliquée, au bout du temps voulu, - par exemple plus de 3 heures - une action de la théophylline, alors que celle du théophyllisilane est terminée.

EXEMPLE 8

Des nanosphères dans l'eau, similaires à celles de l'exemple précédent, ont été obtenues par la copolymérisation de 4 moles de styrène avec 1 mole d'acrylate de méthyle ; elles ne comportaient donc pas de groupes -COOH susceptibles de réagir avec les -NH de la théophylline.

A 100 g de suspension aqueuse de nanosphères de ce copolymère on a ajouté 0,126 g de théophylline et 0,192 g de théophyllisilane : les deux substances ont été adsorbées par les nanosphères.

Comme précédemment, on a déterminé la quantité des principes actifs mis à la disposition de la peau en 3 heures.

Les résultats sont :
20% pour la théophylline
22% pour le théophyllisilane

contre respectivement 10% et 20% dans l'exemple précédent. On voit que le rapport entre les quantités de ces deux substances, absorbables par la peau, a été modifié par le fait de leurs modes de fixation différents sur les nanosphères ; par conséquent, les durées relatives d'action de ces substances sont changées. Ainsi, l'invention permet-elle de modifier la chronologie des actions de substances actives, lorsqu'on fixe celles-ci par des mécanismes différents sur des particules microscopiques de polymère.

EP 0 409 690 B1

EXEMPLE 9

Modification de la chronologie de libération à partir de nanosphères, d'une seule substance active par fixation de celle-ci selon deux mécanismes différents.

(A) - A 100 g de suspension aqueuse de 5 g de nanosphères en le même copolymère qu'à l'exemple 7, comprenant 1 mole d'acide acrylique pour 4 moles de styrène, on ajoute 0,126 g de théophylline qui se fixe ioniquement aux groupes - COOH du copolymère. Ensuite on fait adsorber aux mêmes nanosphères 0,5 g de théophylline.

On détermine la proportion de théophylline qui se trouve à la disposition de la peau, en 3 et en 5 heures, à partir des 100 g de suspension aqueuse de 5 g de nanosphères chargées comme indiqué ci-dessus.

(B) - Parallèlement, des déterminations analogues sont effectuées sur 100 g d'une suspension aqueuse de 5 g de nanosphères en copolymère similaire, comportant des groupes $-COOCH_3$ à la place des -COOH. Ces sphères ne fixent donc pas de théophylline par liaison ionique, mais l'adsorbent. On en a fait adsorber 0,626 g, c'est-à-dire la même quantité totale qu'en A.

Les % de théophylline, pouvant ainsi être absorbées par la peau, sont

|   | en 3 heures | en 5 heures |
|---|---|---|
| A ........... | 15% | 28% |
| B .......... | 18% | 40% |

Il en résulte que, dans le produit A, qui comprend deux ensembles, copolymère + théophylline combinée aux groupes -COOH et copolymère + théophylline adsorbée, la chronologie de l'action sur la peau est différente de celle que donne le produit B portant sur la même quantité de théophylline, mais celle-ci étant seulement et en totalité adsorbée par les nanosphères.

**Revendications**

1. Produit pour applications cutanées, à effets cosmétiques ou/et thérapeutiques, renfermant une ou plusieurs substances actives, portées par des microsphères d'un polymère, dispersées dans un liquide ne dissolvant pas ce polymère et qui comprend au moins deux sortes d'ensembles microsphères-substance active/libérant la substance active en des temps différents, caractérisé en ce que la ou les substances actives sont fixées aux microsphères de polymère par des forces de liaison différentes.

2. Produit suivant la revendication 1, dans lequel les dits ensembles microsphères-substance active diffèrent entre eux par les dimensions des microsphères.

3. Produit suivant la revendication 1 ou 2, dans lequel lesdits ensembles microsphères-substance active diffèrent entre eux par la nature du polymère constituant les microsphères.

4. Produit suivant une des revendications 1 à 3, caractérisé en ce que, auxdites microsphères, plusieurs substances actives sont fixées par adsorption, absorption, liaisons électrostatiques ou chimiques, notamment ioniques, covalentes directes ou/et covalentes indirectes.

5. Produit suivant la revendication 1, caractérisé en ce qu'il comprend une seule substance active, fixée aux dites microsphères par au moins deux sortes de liaisons différentes.

6. Produit suivant une des revendications 1 à 4, caractérisé en ce que les ensembles de microsphères-substance active diffèrent par la composition chimique des substances actives.

7. Produit suivant une des revendications 1 à 5, caractérisé en ce qu'une différence du temps de libération de la substance active est produite par un, composé porteur de groupes fonctionnels, fixé sur les microsphères.

8. Produit suivant une des revendications précédentes, dans lequel les dimensions des microsphères ne dépassent pas 1000 nm, leurs tailles préférées se rangeant entre 50 et 500 nm.

7

9. Produit suivant une des revendications précédentes, dans lequel le liquide de dispersion des microsphères est l'eau, un polyol, ou un éther ou ester de polyol cosmétiquement ou pharmaceutiquement acceptable.

10. Produit suivant une des revendications précédentes, dans lequel le polymère formant les microsphères est un polysaccharide, polyamide, polyalkylène, poly-arylalkylène, polyalkylidène ou polysilicone.

11. Produit suivant une des revendications précédentes, dans lequel la teneur en microsphères est de 0,1 à 10% en poids, et en particulier de 0,3 à 3%, et la teneur en substance active est de 0,01 à 90% en poids.

12. Produit suivant une des revendications précédentes, dans lequel la ou les substances actives sont des hydratants, régénérateurs, réparateurs, amincissant, antiradicaux libres, antiglycosylants, protecteurs des U.V., activateurs de bronzage, colorants ou désodorisants.

13. Produit suivant une des revendications précédentes, dans lequel la ou les substances actives sont des vitamines, hormones, enzymes, vasodilatateurs, vasoconstricteurs, anti-inflammatoires, antiseptiques, cholagogues, diurétiques, antiallergiques ou neuroleptiques.

14. Produit suivant une des revendications précédentes, qui renferme un composé auxiliaire, miscible avec le liquide de dispersion des microsphères, en particulier un polyol, polysaccharide, mucopolysaccharide, ou leurs dérivés siliciés , ou/et un tensioactif.

15. Procédé de préparation d'un produit suivant une des revendications 1 à 14, qui consiste à mélanger des microsphères de polymère avec une ou plusieurs substances actives, dans un liquide de dispersion ne dissolvant pas le polymère des microsphères, carctérisé en ce que l'on utilise des microsphères en un polymère portant des groupes fonctionnels, susceptibles de se combiner à au moins une des substances actives, tandis que ce polymère continue à adsorber des substances actives.

16. Procédé suivant la revendication 15, caractérisé en ce que,des groupes fonctionnels voulus sont formés sur les microsphères de polymère par l'action d'un réactif, après quoi on ajoute la substance active devant être fixée à ces groupes.

17. Procédé suivant la revendication 15, caractérisé en ce que l'on emploie une substance active dont une partie est adsorbée par les microsphères et une partie fixée par les groupes fonctionnels du polymère.

18. Procédé suivant une des revendications 15 à 17, caractérisé en ce que les groupes fonctionnels sont des groupes carboxyliques ou/et amino.

## Patentansprüche

1. Produkt für kutane Anwendungen mit kosmetischen und/oder therapeutischen Wirkungen, das eine oder mehrere aktive Substanzen enthält, mit Mikrokugeln aus einem Polymeren als Trägermaterial, welche in einer Flüssigkeit dispergiert sind, die dieses Polymere nicht auflöst und die mindestens zwei Arten der Komplexe aus Mikrokugeln-aktiver Substanz enthält, die die aktive Substanz zu verschiedenen Zeiten freisetzen, dadurch gekennzeichnet, daß die aktive Substanz oder die aktiven Substanzen durch verschiedene Bindungskräfte an die Mikrokugeln aus dem Polymeren gebunden sind.

2. Produkt nach Anspruch 1, bei dem die genannten komplexe aus Mikrokugeln-aktiver Substanz sich voneinander durch die Dimensionen der Mikrokugeln unterscheiden.

3. Produkt nach Anspruch 1 oder 2, bei dem die genannten komplexe aus Mikrokugeln-aktiver Substanz sich durch die Art des Polymeren unterscheiden, aus dem die Mikrokugeln bestehen.

4. Produkt nach einem der Ansprüche 1 bis 3, dadurch gekennzeichnet, daß an die genannten Mikrokugeln mehrere aktive Substanzen gebunden sind durch Adsorption, Absorption, elektrostatische oder chemische Bindungen, insbesondere ionische, kovalente direkte oder/und kovalente indirekte Bindungen.

5. Produkt nach Anspruch 1, dadurch gekennzeichnet, daß es eine einzige aktive Substanz enthält, die an die genannten Mikrokugeln durch mindestens zwei Arten verschiedener Bindungen gebunden ist.

6. Produkt nach einem der Ansprüche 1 bis 4, dadurch gekennzeichnet, daß die Komplexe aus Mikrokugeln-aktiver Substanz sich durch die chemische Zusammensetzung der aktiven Substanzen unterscheidet.

7. Produkt nach einem der Ansprüche 1 bis 5, dadurch gekennzeichnet, daß ein Unterschied in der Freisetzungszeit der aktiven Substanz erzeugt wird durch eine Verbindung als Träger funktioneller Gruppen, welche an die Mikrokugeln gebunden sind.

8. Produkt nach einem der vorausgegangenen Ansprüche, bei dem die Dimensionen der Mikrokugeln 1000 nm nicht überschreiten und ihre bevorzugten Größen im Bereich zwischen 50 und 500 nm liegen.

9. Produkt nach einem der vorausgegangenen Ansprüche, bei dem die Dispersionsflüssigkeit für die Mikrokugeln Wasser, ein Polyol oder Ether oder ein pharmazeutisch verträglicher Polyolester ist.

10. Produkt nach einem der vorausgegangenen Ansprüche, bei dem das Polymere, welches die Mikrokugeln bildet, ein Polysaccharid, Polyamid, Polyalkylen, Polyarylalkylen, Polyalkyliden oder Polysilicon ist.

11. Produkt nach einem der vorausgegangenen Ansprüche, bei dem der Gehalt an Mikrokugeln 0,1 bis 10 Gew.-%, insbesondere 0,3 bis 3 Gew.-%, beträgt und der Gehalt an aktiver Substanz 0,01 bis 90 Gew.-% beträgt.

12. Produkt nach einem der vorausgegangenen Ansprüche, bei dem die aktive Substanz oder die aktiven Substanzen feuchtigkeitsspendende Mittel, Verbesserungsmittel, Reparaturmittel, Verjüngungsmittel, Mittel gegen freie Radikale, Antiglycosylierungsmittel, U.V.-Schutzmittel, Bräunungsaktivatoren, Farbstoffe oder Desodorantien sind.

13. Produkt nach einem der vorausgegangenen Ansprüche, bei dem die aktive Substanz oder die aktiven Substanzen Vitamine, Hormone, Enzyme, Vasodilatatoren, Vasokonstriktoren, anti-inflammatorische Mittel, Antiseptika, Cholinergika, Diuretika, Antiallergika oder Neuroleptika sind.

14. Produkt nach einem der vorausgegangenen Ansprüche, enthaltend einen Hilfsstoff, der mit der Dispersionsflüssigkeit für die Mikrokugeln mischbar ist, insbesondere Polyol, Polysaccharid, Mucopolysaccharid oder ihre Siliciumderivate oder/und ein oberflächenaktives Mittel.

15. Verfahren zur Herstellung eines Produktes, nach einem der Ansprüche 1 bis 14, welches umfaßt Vermischen der Mikrokugeln aus dem Polymeren mit einer oder mehreren aktiven Substanzen in einer Dispersionsflüssigkeit, die das Polymere der Mikrokugeln nicht auflöst, dadurch gekennzeichnet, daß Mikrokugeln aus einem Polymeren verwendet werden, welches funktionelle Gruppen trägt, die leicht mit mindestens einer der aktiven Substanzen eine Bindung eingehen können, während dieses Polymere weiterhin aktive Substanzen adsorbiert.

16. Verfahren nach Anspruch 15, dadurch gekennzeichnet, daß die genannten funktionellen Gruppen auf den Mikrokugeln aus dem Polymeren durch eine Reaktion gebildet werden, bei der die aktive Substanz hinzugegeben wird bevor sie an diese Gruppen gebunden wird.

17. Verfahren nach Anspruch 15, dadurch gekennzeichnet, daß eine aktive Substanz eingesetzt wird, von der ein Teil von den Mikrokugeln adsorbiert und ein Teil durch die funktionellen Gruppen des Polymeren gebunden wird.

18. Verfahren nach einem der Ansprüche 15 bis 17, dadurch gekennzeichnet, daß die funktionellen Gruppen Carboxyl- oder/und Aminogruppen sind.

## Claims

1. A product for cutaneous applications with cosmetic and/or therapeutic effects, including one or more active substances, carried by microspheres of a polymer, dispersed in a liquid which does not dissolve this polymer and which comprises at least two kinds of microspheres-active substance sets, releasing the active substance at different times; characterised in that the active substance(s) is (are) bound to the microspheres of polymer by different bond strengths.

2. The product according to claim 1 wherein said sets of microspheres-active substance differ from each other by the size of the microspheres

3. The product according to claim 1 or 2 wherein said sets of microspheres-active substance differ from each other by the nature of the polymer constituting the microspheres.

4. The product according to one of claims 1 to 3, characterized in that, to said microspheres, several active substances are bound by adsorption, absorption, electrostatic or chemical bonds in particular ionic, direct covalent and/or indirect covalent.

5. The product according to claim 1, characterized in that it comprises a single active substance bound to the microspheres by at least two kinds of different bonds.

6. The product according to one of claims 1 to 4, characterized in that said sets of microspheres-active substance differ by the chemical composition of the active substances.

7. The product according to one of claims 1 to 5 characterized in that a difference in the times the active substance is released at is produced by a compound carrying functional groups bound to the microspheres.

8. The product according to one of the preceding claims, in which the size of the microspheres does not exceed 1000 nm, their prefered sizes ranging from 50 to 500 nm.

9. The product according to one of the preceding claims, in which the microspheres dispersion liquid is water, a polyol, or an ether or ester of a cosmetically or pharmaceutically acceptable polyol.

10. The product according to one of the preceding claims, in which the polymer making up the microspheres is a polysaccharide, polyamide, polyalkylene, polyarylalkylene, polyalkylidene or polysilicone.

11. The product according to one of the preceding claims, in which content in microspheres ranges from 0,1 to 10% in weight and, in particular, from 0,3 to 3%, and content in active substance ranges from 0,01 to 90% in weight.

12. The product according to one of the preceding claims, in which the active substance(s) is or are hydrators, restructurers, repairers, slimming products, anti-free radical agents, antiglycosylants, anti-ultraviolet agents, tanning activators, dyes or deodorants.

13. The product according to one of the preceding claims in which the active substance(s) is or are vitamins, hormones, enzymes, vasodilators, vasoconstrictors, anti-inflammatory, antiseptics, cholagogs, diuretics, anti-allergic or neuroleptic agents.

14. Product according to one of the preceding claims, containing a secondary compound, miscible with the microspheres dispersion liquid, in particular a polyol, polysaccharide, mucopolysaccharide, or their silicated derivatives, and/or a surfactant.

15. Process for the preparation of a product according to one of claims 1 to 14, which consists in mixing microspheres of polymer with one or several active substances, in a dispersion liquid which does not dissolve the polymer of the microspheres, characterized in that are used microspheres of a polymer carrying functional groups able to combine with at least one of the active substances, while the polymer keeps on adsorbing active substances.

16. Process according to claim 15, characterized in that desired functional groups are formed on the microspheres of polymer by the action of a reagent, afterwards the active substance to be bound to these groups is added.

17. Process according to claim 15, characterized in that is used an active substance a portion of which is adsorbed by the microspheres and a portion bound by the functional groups of the polymer.

18. Process according to one of claims 15 to 17, characterized in that the functional groups are carboxylic or/and amino groups.